(19) Europäisches Patentamt
European Patent Office
Office européen des brevets

(11) EP 0 769 493 B1

(12) FASCICULE DE BREVET EUROPEEN

(45) Date de publication et mention de la délivrance du brevet:
**18.09.2002 Bulletin 2002/38**

(51) Int Cl.[7]: **C07D 233/32**

(21) Numéro de dépôt: **96402172.9**

(22) Date de dépôt: **11.10.1996**

# (54) Procédé de préparation de (méth)acrylates d'alkylimidazolidone

Verfahren zur Herstellung von Alkylimidazolidinon(meth)acrylaten

Process for the preparation of alkylimidazolidinone (meth)acrylates

(84) Etats contractants désignés:
**AT BE CH DE DK ES FI FR GB GR IE IT LI LU NL PT SE**

(30) Priorité: **17.10.1995 FR 9512150**

(43) Date de publication de la demande:
**23.04.1997 Bulletin 1997/17**

(73) Titulaire: **Atofina**
**92800 Puteaux (FR)**

(72) Inventeurs:
- **Riondel, Alain**
  **57600 Forbach (FR)**
- **Paul, Jean-Michel**
  **57010 Metz (FR)**

(74) Mandataire: **Rieux, Michel**
**Atofina**
**D.C.R.D./D.P.I.**
**4, Cours Michelet,**
**La Défense 10**
**92091 Paris la Défense Cedex (FR)**

(56) Documents cités:
**EP-A- 0 226 501**
**EP-A- 0 236 994**
**FR-A- 1 327 383**
**US-A- 2 871 223**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 769 493 B1

Printed by Jouve, 75001 PARIS (FR)

## Description

La présente invention porte sur un procédé de fabrication d'un composé de formule :

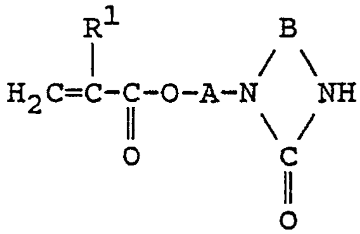

(I)

dans laquelle :

- $R^1$ représente hydrogène ou méthyle ; et
- A et B représentent chacun indépendamment un groupe alkylène à chaîne droite ou ramifiée, ayant de 2 à 5 atomes de carbone.

Ces composés de formule (I) sont connus pour leur rôle dans la constitution de polymères utiles comme revêtements et adhésifs, pour le traitement du papier et des textiles, notamment par le brevet américain US-A-2 871 223, ainsi que pour leurs utilisations comme agents de traitement du cuir, et dans la production de peintures en émulsion. Le méthacrylate d'éthylimidazolidone (MEIO) est principalement utilisé en tant que promoteur d'adhésion humide.

Le brevet américain précité US-A-2 871 223 décrit la préparation du MEIO par réaction de la 1-(2-hydroxyéthyl)-imidazolidyl-2-one (HEIO) avec le chlorure de méthacryloyle. Il est connu de faire réagir une amine sur un anhydride (voir EP-A-226 501).

Le MEIO est cependant classiquement préparé par transestérification. Ainsi, les demandes de brevets européens EP-A-0 236 994, EP-A-0 433 135, EP-A-0 453 638, EP-A-0 571 851, EP-A-0 619 309, et la demande de brevet français n° 94-13848 du 18 novembre 1994 au nom de la Société déposante, décrivent la préparation d'un composé de la formule (I) définie ci-dessus par réaction d'au moins un (méth)acrylate de formule (IV) :

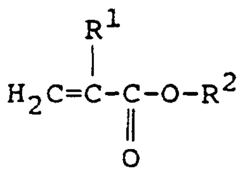

(IV)

dans laquelle :

- $R^1$ représente H ou $CH_3$ ; et
- $R^2$ représente un groupe alkyle en $C_1$-$C_4$,

avec un alcool hétérocyclique de formule (III) :

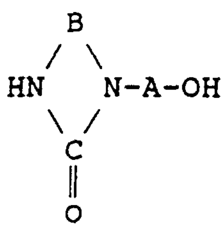

(III)

dans laquelle A et B ont les significations précitées, en présence de différents catalyseurs ou systèmes catalytiques.

Ces différents procédés de transestérification conduisent à l'obtention d'un produit de formule (I) sous une

forme qui ne convient que pour certaines applications. C'est notamment le cas du MEIO qui est obtenu sous la forme d'une solution dans le méthacrylate de méthyle (MAM).

La Société déposante a maintenant découvert que la réaction de l'alcool (III) avec l'anhydride (méth)acrylique permet d'obtenir, après hydrolyse de l'anhydride (méth)acrylique engagé en excès par rapport à l'alcool (III) un composé de formule (I) en solution dans l'acide (méth)acrylique et l'eau, convenant pour de nouvelles applications.

La présente invention a donc pour objet un procédé de fabrication d'un composé de la formule (I) telle que définie ci-dessus, caractérisé par le fait que l'on fait réagir un anhydride de formule (II) :

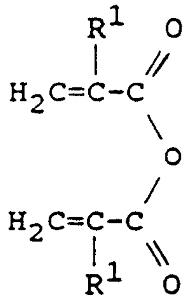

(II)

dans laquelle $R^1$ est tel que défini ci-dessus,

avec un alcool hétérocyclique de la formule (III) également telle que définie ci-dessus.

La réaction ci-dessus peut être conduite en présence d'au moins un catalyseur d'estérification. Comme catalyseurs d'estérification, on peut citer, par exemple, les acides de Brönsted, tels que l'acide sulfurique, les acides alkyl et aryl sulfoniques, les résines acides à groupements acide sulfonique ; les acides de Lewis, tels que le trifluorure de bore, le chlorure de zinc ; ou encore des catalyseurs basiques tels que des amines tertiaires comme la triéthylamine, le 1-méthylimidazole ; ainsi que les formes supportées de ces catalyseurs sur des supports solides, par exemple sur alumine, charbon actif, silice, résine acide, etc.

La quantité de catalyseur(s) utilisée pour la mise en oeuvre du procédé selon l'invention est généralement comprise entre 0,1 et 15% environ en moles, de préférence entre 0,5 et 5% environ en moles, par rapport à l'alcool (III).

La réaction du procédé selon l'invention est avantageusement effectuée avec un rapport molaire anhydride (II) / alcool hétérocyclique (III) généralement compris entre 1,2 et 10 environ, de préférence entre 1,5 et 3.

De plus, cette réaction est, de préférence, effectuée sous bullage d'air en présence d'au moins un inhibiteur de polymérisation, utilisé, par exemple, à raison de 0,01 à 0,35% en poids sur la base du poids de la charge réactionnelle. Comme exemples d'inhibiteurs de polymérisation utilisables, on peut citer notamment la phénothiazine, l'éther monométhylique de l'hydroquinone, le 2,6-ditertiobutylparacrésol, l'hydroquinone, la paraphénylènediamine et leurs mélanges en toutes proportions.

La réaction selon l'invention est généralement effectuée à une température comprise entre 20 et 120°C, et, plus particulièrement, entre 30 et 90°C, et à la pression atmosphérique. La durée de la réaction selon l'invention, dépendant évidemment de conditions réactionnelles, telles que la température et la quantité de catalyseur(s) utilisé (s), ainsi que de la nature des réactifs mis en oeuvre, est généralement comprise entre 2 et 15 heures environ.

Après la réaction, l'excès d'anhydride (méth)acrylique est hydrolysé en acide (méth)acrylique par ajout d'eau et chauffage entre 25 et 70°C, et, de préférence, entre 30 et 50°C, afin d'obtenir le composé (I) en solution dans l'eau et l'acide (méth)acrylique.

L'hydrolyse peut être suivie par dosage potentiométrique de l'acide (méth)acrylique formé ; elle dure généralement de 3 à 10 heures.

Les exemples suivants illustrent l'invention sans toutefois la limiter. Dans ces exemples, les pourcentages sont indiqués en poids sauf indication contraire.

<u>Exemple 1</u>

<u>Etape 1</u>

Dans un réacteur en verre, à double enveloppe, équipé d'une sonde de mesure de la température (en pied), d'un agitateur mécanique du type ancre à vitesse variable, et d'une canne plongeante pour l'introduction d'air, on introduit 178,1 g d'anhydride méthacrylique, 99,71 g d'HEIO, 0,77 g de 2,6-ditertiobutylparacrésol et 0,075 g d'éther monométhylique de l'hydroquinone en tant que stabilisants et 2,9 g de 1-méthylimidazole en tant que catalyseur.

On porte le mélange à 50°C sous bullage d'air à la pression atmosphérique.

Le suivi de la réaction est réalisé par chromatographie sur couche mince. La durée de la réaction est de 5 heures.

Etape 2

L'anhydride méthacrylique excédentaire est ensuite hydrolysé en acide méthacrylique à 50°C pendant 7 heures, la quantité d'eau introduite étant de 96 g.
Le suivi de cette étape est assuré par dosage potentiométrique de l'acide méthacrylique formé.
On obtient ainsi un produit qui se présente sous la forme d'un liquide limpide, légèrement jaune, ayant la composition suivante :

| | |
|---|---|
| MEIO | 31,5% |
| Acide méthacrylique (AMA) | 32% |
| Eau | 20% |
| HEIO | 0,3% |
| 2,6-Ditertiobutylparacrésol | 2500 ppm |

La conversion de l'HEIO, le rendement en MEIO et la sélectivité en MEIO sont déterminés par les équations suivantes d'après l'analyse par chromatographie en phase liquide HPLC du brut réactionnel.

Conversion de l'HEIO (%)

$$C = \frac{\text{Nombre de moles d'HEIO converti}}{\text{Nombre de moles d'HEIO de départ}} \times 100$$

Rendement en MEIO (%)

$$R = \frac{\text{Nombre de moles de MEIO formé}}{\text{Nombre de moles d'HEIO de départ}} \times 100$$

Sélectivité en MEIO (%)

$$S = \frac{\text{Nombre de moles de MEIO formé}}{\text{Nombre de moles d'HEIO converti}} \times 100$$

Exemple 2

On a reproduit l'Exemple 1, excepté que l'on a conduit la réaction de l'Etape 1 à 82°C, sans catalyseur et en utilisant comme stabilisant, 0,18 g de phénothiazine.
Les résultats des essais effectués sont rapportés dans le Tableau ci-après.

Tableau

| Exemple | Réaction de l'Étape 1 | | Analyse HPLC du brut de l'Etape 2 (%) | | | C (%) | R (%) | S (%) |
|---|---|---|---|---|---|---|---|---|
| | Température (°C) | Durée (h) | AMA | HEIO | MEIO | | | |
| 1 | 50 | 5 | 32 | 0,3 | 31,5 | 99 | 67,6 | 66 |
| 2 | 82 | 6 | 30,5 | 4,32 | 33,5 | 89,5 | 53,3 | 59,5 |

**Revendications**

1. Procédé de fabrication d'un composé de formule :

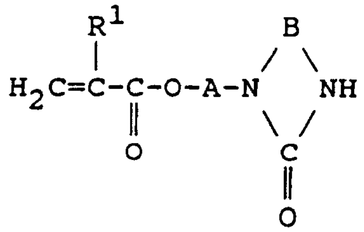

(I)

dans laquelle :

- $R^1$ représente hydrogène ou méthyle ; et
- A et B représentent chacun indépendamment un groupe alkylène à chaîne droite ou ramifiée, ayant de 2 à 5 atomes de carbone,

**caractérisé par le fait que** l'on fait réagir un anhydride de formule (II) :

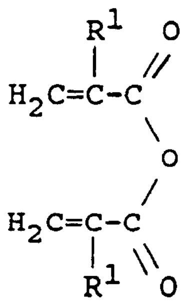

(II)

dans laquelle $R^1$ est tel que défini ci-dessus,
avec un alcool hétérocyclique de formule (III) :

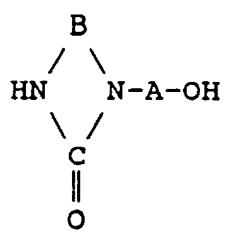

(III)

dans laquelle A et B ont les significations précitées.

2. Procédé selon la revendication 1, **caractérisé par le fait que** l'on conduit la réaction en présence d'au moins un catalyseur d'estérification choisi notamment parmi les acides de Brönsted, tels que l'acide sulfurique, les acides alkyl et aryl sulfoniques, les résines acides à groupements acide sulfonique ; les acides de Lewis, tels que le trifluorure de bore, le chlorure de zinc ; ; ou encore des catalyseurs basiques tels que des amines tertiaires comme la triéthylamine, le 1-méthylimidazole ; ainsi que les formes supportées de ces catalyseurs sur des supports solides, par exemple sur alumine, charbon actif, silice, résine acide.

3. Procédé selon la revendication 2, **caractérisé par le fait que** l'on introduit le ou les catalyseurs à raison de 0,1 à 15% en moles par rapport à l'alcool (III), en particulier à raison de 0,5 à 5% en moles par rapport à l'alcool (III).

**4.** Procédé selon l'une des revendications 1 à 3, **caractérisé par le fait que** l'on utilise les réactifs dans un rapport molaire anhydride (II) / alcool (III) compris entre 1,2 et 10, de préférence entre 1,5 et 3.

**5.** Procédé selon l'une des revendications 1 à 4, **caractérisé par le fait que** l'on conduit la réaction en présence d'au moins un inhibiteur de polymérisation utilisé notamment à raison de 0,01 à 0,35% en poids sur la base du poids de la charge réactionnelle.

**6.** Procédé selon la revendication 5, **caractérisé par le fait que** l'inhibiteur de polymérisation est choisi parmi la phénothiazine, l'éther monométhylique de l'hydroquinone, le 2,6-ditertiobutylparacrésol, l'hydroquinone, la paraphénylènediamine et leurs mélanges en toutes proportions.

**7.** Procédé selon l'une des revendications 1 à 6, **caractérisé par le fait que** l'on conduit la réaction à une température comprise entre 20 et 120°C, notamment entre 30 et 90°C.

**8.** Procédé selon l'une des revendications 1 à 7, **caractérisé par le fait que** l'on conduit la réaction pendant une durée de 2 à 15 heures.

**9.** Procédé selon l'une des revendications 1 à 8, **caractérisé par le fait qu'**après la réaction, on hydrolyse l'excès d'anhydride (méth)acrylique en acide (méth)acrylique par ajout d'eau et chauffage entre 25 et 70°C, de préférence entre 30 et 50°C, afin d'obtenir le composé (I) en solution dans l'eau et l'acide (méth)acrylique.

**10.** Procédé selon la revendication 9, **caractérisé par le fait que** l'on conduit l'hydrolyse pendant une durée de 3 à 10 heures.

**Claims**

**1.** Process for the manufacture of a compound of formula:

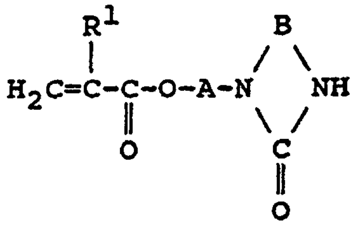

(I)

in which:

- $R^1$ represents hydrogen or methyl; and
- A and B each independently represent a straight- or branched-chain alkylene group having from 2 to 5 carbon atoms,

**characterized in that** an anhydride of formula (II):

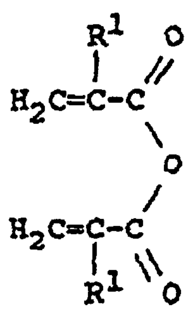

(II)

in which $R^1$ is as defined above,
is reacted with a heterocyclic alcohol of formula (III):

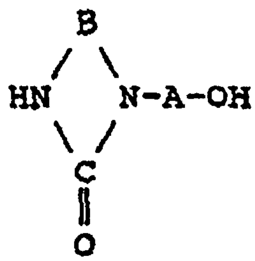

(III)

in which A and B have the abovementioned meanings.

2. Process according to Claim 1, **characterized in that** the reaction is carried out in the presence of at least one esterification catalyst chosen in particular from Brönsted acids, such as sulphuric acid, alkyl- and arylsulphonic acids or acidic resins containing sulphonic acid groups; Lewis acids, such as boron trifluoride or zinc chloride; or alternatively basic catalysts, such as tertiary amines, for example triethylamine or 1-methylimidazole; as well as the supported forms of these catalysts on solid supports, for example on alumina, active charcoal, silica or acidic resin.

3. Process according to Claim 2, **characterized in that** the catalyst or catalysts is/are introduced in the proportion of 0.1 to 15% on a molar basis with respect to the alcohol (III), in particular in the proportion of 0.5 to 5% on a molar basis with respect to the alcohol (III).

4. Process according to one of Claims 1 to 3, **characterized in that** the reactants are used in an anhydride (II)/alcohol (III) molar ratio of between 1.2 and 10, and preferably between 1.5 and 3.

5. Process according to one of Claims 1 to 4, **characterized in that** the reaction is carried out in the presence of at least one polymerization inhibitor used in particular in the proportion of 0.01 to 0.35% by weight with respect to the weight of the reaction charge.

6. Process according to Claim 5, **characterized in that** the polymerization inhibitor is chosen from phenothiazine, hydroquinone monomethyl ether, 2,6-di-tert-butyl-para-cresol, hydroquinone, paraphenylenediamine and their mixtures in all proportions.

7. Process according to one of Claims 1 to 6, **characterized in that** the reaction is carried out at a temperature of between 20 and 120°C, in particular between 30 and 90°C.

8. Process according to one of Claims 1 to 7, **characterized in that** the reaction is carried out for a duration of 2 to 15 hours.

9. Process according to one of Claims 1 to 8, **characterized in that**, after the reaction, the excess (meth)acrylic anhydride is hydrolysed to (meth)acrylic acid by addition of water and heating between 25 and 70°C, preferably between 30 and 50°C, in order to obtain the compound (I) in solution in water and (meth)acrylic acid.

10. Process according to Claim 9, **characterized in that** the hydrolysis is carried for a duration of 3 to 10 hours.

**Patentansprüche**

1. Verfahren zur Herstellung einer Verbindung der Formel:

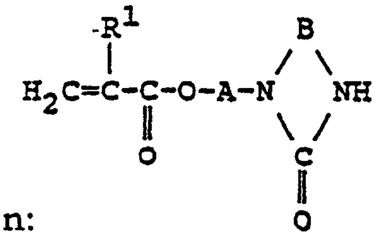

(I),

n:

worin bedeuten:

- $R^1$ Wasserstoff oder Methyl, und
- A und B jeweils unabhängig voneinander eine geradkettige oder verzweigte Alkylengruppe mit 2 bis 5 Kohlenstoffatomen,

**dadurch gekennzeichnet, daß** ein Anhydrid der Formel (II):

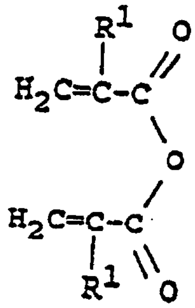

(II),

in der $R^1$ die oben angegebene Bedeutung aufweist, mit einem heterocyclischen Alkohol der Formel (III) umgesetzt wird:

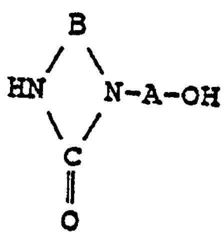

(III),

wobei A und B die oben angegebenen Bedeutungen aufweisen.

**2.** Verfahren nach Anspruch 1, **dadurch gekennzeichnet, daß** die Umsetzung in Gegenwart mindestens eines Veresterungskatalysators durchgeführt wird, der insbesondere unter den Brönsted-Säuren, wie Schwefelsäure, Alkyl- und Arylsulfonsäuren und sauren Harzen mit Sulfonsäuregruppen; Lewis-Säuren, wie Bortrifluorid und Zinkchlorid; oder basischen Katalysatoren, wie tertiären Aminen, beispielsweise Triethylamin und 1-Methylimidazol; sowie den Ausführungsformen, nach denen diese Katalysatoren auf festen Trägern vorliegen, beispielsweise auf Aluminiumoxid, Aktivkohle, Kieselsäure oder einem sauren Harz, ausgewählt ist.

**3.** Verfahren nach Anspruch 2, **dadurch gekennzeichnet, daß** der Katalysator oder die Katalysatoren in Mengenanteilen von 0,1 bis 15 Mol-%, bezogen auf den Alkohol (III), und insbesondere in einer Menge von 0,5 bis 5 Mol-%, bezogen auf den Alkohol (III), eingearbeitet werden.

**4.** Verfahren nach einem der Ansprüche 1 bis 3, **dadurch gekennzeichnet, daß** die Reaktanten in einem Molverhältnis Anhydrid (II)/Alkohol (III) im Bereich von 1,2 und 10 und vorzugsweise 1,5 bis 3 verwendet werden.

5. Verfahren nach einem der Ansprüche 1 bis 4, **dadurch gekennzeichnet, daß** die Umsetzung in Gegenwart mindestens eines Polymerisationsinhibitors durchgeführt wird, der insbesondere in einem Mengenanteil von 0,01 bis 0,35 Gew.-%, bezogen auf das Gewicht des Reaktionsgemisches, verwendet wird.

6. Verfahren nach Anspruch 5, **dadurch gekennzeichnet, daß** der Polymerisationsinhibitor unter Phenothiazin, Hydrochinonmonomethylether, 2,6-Di-t-butyl-p-kresol, Hydrochinon, p-Phenylendiamin und deren Gemischen in beliebigen Anteilen ausgewählt ist.

7. Verfahren nach einem der Ansprüche 1 bis 6, **dadurch gekennzeichnet, daß** die Umsetzung bei einer Temperatur im Bereich von 20 bis 120 °C und insbesondere 30 bis 90 °C durchgeführt wird.

8. Verfahren nach einem der Ansprüche 1 bis 7, **dadurch gekennzeichnet, daß** die Umsetzung während einer Zeitspanne von 2 bis 15 h durchgeführt wird.

9. Verfahren nach einem der Ansprüche 1 bis 8, **dadurch gekennzeichnet, daß** nach der Umsetzung das überschüssige (Meth)-acrylsäureanhydrid zu (Meth)acrylsäure hydrolysiert wird, indem Wasser zugegeben und das Gemisch auf 25 bis 70 °C und vorzugsweise 30 bis 50 °C erwärmt wird, wodurch die Verbindung (I) gelöst in Wasser und (Meth)acrylsäure erhalten wird.

10. Verfahren nach Anspruch 9, **dadurch gekennzeichnet, daß** die Hydrolyse während einer Zeitspanne von 3 bis 10 h durchgeführt wird.